(19) Europäisches Patentamt
European Patent Office
Office européen des brevets

(11) EP 2 011 526 B1

(12) EUROPEAN PATENT SPECIFICATION

(45) Date of publication and mention of the grant of the patent:
**27.04.2011 Bulletin 2011/17**

(51) Int Cl.:
***A61K 9/70*** *(2006.01)*

(21) Application number: **08252296.2**

(22) Date of filing: **04.07.2008**

(54) **Patch and patch preparation**

Flicken und Flickenherstellung

Patch et préparation de patch

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **05.07.2007 JP 2007176844**

(43) Date of publication of application:
**07.01.2009 Bulletin 2009/02**

(73) Proprietor: **Nitto Denko Corporation**
**Ibaraki-shi**
**Osaka 567-8680 (JP)**

(72) Inventors:
- **Hamada, Atsushi c/o Nitto Denko Corporation Ibaraki-shi, Osaka 567-8680 (JP)**
- **Kasahara, Tsuyoshi c/o Nitto Denko Corporation Ibaraki-shi, Osaka 567-8680 (JP)**
- **Ishikura, Jun c/o Nitto Denko Corporation Ibaraki-shi, Osaka 567-8680 (JP)**
- **Funahashi, Miki c/o Nitto Denko Corporation Ibaraki-shi, Osaka 567-8680 (JP)**

(74) Representative: **Duckworth, Timothy John**
**J.A. Kemp & Co.**
**14 South Square**
**Gray's Inn**
**London WC1R 5JJ (GB)**

(56) References cited:
**EP-A- 0 781 553**
**EP-A- 1 970 079**
**EP-A- 2 002 824**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 011 526 B1

## Description

### TECHNICAL FIELD OF THE INVENTION

The present invention relates to a patch and a patch preparation.

### BACKGROUND OF THE INVENTION

Conventionally, a patch is developed, which comprises an adhesive layer formed on one surface of a support. Such adhesive layer is required to certainly fix a patch on the skin surface. However, when the skin adhesive force is too high, the skin may be irritated during detachment of the patch from the skin. As a method of decreasing such skin stimulation, an adhesive composition may contain an organic liquid component. In addition, when the adhesive layer is a patch preparation containing a drug, the adhesive composition may contain an organic liquid component in order to control the releaseability of the drug from the adhesive layer or to maintain the drug stably in the adhesive layer.

To achieve such object, an adhesive layer is suggested, wherein an acrylic polymer is crosslinked to maintain an organic liquid component. However, a chemical component such as a certain kind of drug sometimes shows low solubility in acrylic polymers. As such, a patch based on conventional acrylic polymers is not sufficiently satisfactory from the practical aspect.

On the other hand, an attempt has been made to increase the cohesive strength of an adhesive layer containing a non-acrylic polymer, e.g., polyisobutylene, by adding polyisobutylene with a higher molecular weight to the adhesive layer, thereby to maintain an organic liquid component. However, while such adhesive layer can maintain an organic liquid component to a certain level, an adhesive layer containing a large amount of an organic liquid component shows decreased cohesive strength. As a result, when a patch is applied to the skin, the adhesive layer tends to remain on the skin or the adhesive force tends to decrease. Thus, this type of patch is not sufficiently satisfactory.

Accordingly, there is a demand for a patch and a patch preparation, wherein an adhesive layer containing a non-acrylic polymer is crosslinked. The related technical references are as follows.

JP-A-10-151185 discloses a crosslinked adhesive layer containing 30 - 100 parts by weight of a rubber component having a functional group and 0 - 70 parts by weight of other rubber, as well as 20 - 80 parts by weight of liquid oil compatible with the rubber component, relative to 100 parts by weight of the rubber component. However, this reference does not disclose addition, to the adhesive layer, of less than 30 wt% of a liquid rubber component having a functional group in a molecule, relative to the total weight of a synthetic rubber component (solid at 25˚C) except polyisobutylene and the liquid rubber component.

JP-A-3-127727 discloses an adhesive layer comprising an adhesive mainly constituted with a non-acrylic compound and having a glass transition temperature of not more than -71˚C, a reinforcing filler and a pharmaceutical agent. However, this reference does not specifically disclose co-presence of a liquid rubber component having a functional group and other synthetic rubber component in an adhesive layer, and crosslinking of the adhesive layer.

In Example 4 thereof describes an adhesive layer containing liquid polybutadiene containing a hydroxyl group on both terminals, liquid polybutadiene containing 2 - 2.5 functional NCO groups, dibutyltinacetate as a catalyst for crosslinking and the like. However, in this Example, only a liquid rubber component having a functional group is contained as a rubber component, and other synthetic rubber component is not co-present in the adhesive layer. Moreover, an adhesive sheet having such an adhesive layer is reported to have shown an adhesive sticking out by 3 mm or below from one edge when applied to a human body, even though it does not contain an organic liquid component. This reveals that the patch of this Example is not sufficiently satisfactory from the practical aspect.

EP-A-0781553 describes a patch comprising an antiphlogistic having carboxylic groups in its molecule, 1-menthol and a metal salt of a fatty acid. EP-A-1970079 describes an adhesive preparation having an adhesive layer which comprises a first synthetic rubber having a viscosity average molecular weight of 1,600,000-6,500,000, a drug, an organic liquid component and a tackifier.

### Disclosure of the Invention

### Problems to be Solved by the Invention

In view of the above, the present invention aims to provide a patch and a patch preparation that do not require an acrylic polymer, and are capable of maintaining a large amount of an organic liquid component in an adhesive layer.

### Means of Solving the Problems

The present inventors have conducted intensive studies and found that an adhesive layer can maintain a large

amount of an organic liquid component when the adhesive layer contains a synthetic rubber component other than polyisobutylene and solid at 25˚C, a liquid rubber component having a crosslinkable functional group in the molecule of the liquid rubber component, and an organic liquid component, the adhesive layer is crosslinked, and the liquid rubber component is contained in the adhesive layer at a given ratio. Moreover, they have unexpectedly found that a patch having such an adhesive layer is highly practical in terms of skin adhesiveness and adhesive residue, which resulted in the completion of the present invention.

Accordingly, the present invention provides the following.

(1) A patch comprising a support and an adhesive layer provided on at least one surface of the support, wherein the adhesive layer comprises a synthetic rubber component other than polyisobutylene and solid at 25˚C; a liquid rubber component having a crosslinkable functional group in the molecule of the liquid rubber component, and an organic liquid component; wherein the adhesive layer is crosslinked, and the liquid rubber component is contained in a proportion of less than 30 wt% relative to the total weight of the synthetic rubber component solid at 25˚C and the liquid rubber component.
(2) The patch of (1), wherein the adhesive layer further comprises a tackifier.
(3) The patch of (1) or (2), wherein the liquid rubber component is a liquid isoprene rubber.
(4) The patch of any one of (1) to (3), wherein the liquid rubber component contains, in the molecule of the liquid rubber component, 3 or more crosslinkable functional groups.
(5) The patch of any one of (1) to (3), wherein the synthetic rubber component solid at 25˚C is at least one kind selected from the group consisting of a polyisoprene rubber, a polybutadiene rubber and a styrene-diene-styrene block copolymer.
(6) The patch of any one of (1) to (5), wherein the adhesive layer is crosslinked with a crosslinking agent, and the ratio of the total number (A) of the functional groups of the crosslinking agent in the adhesive layer to the total number (B) of the functional groups of the liquid rubber component in the adhesive layer, (((A)/(B))x100)[%], is not less than 50%.
(7) The patch of any one of (1) to (6), wherein the organic liquid component is contained in a proportion of not less than 10 wt% relative to the total weight of the adhesive layer (when the adhesive layer contains a crosslinking agent, the weight of the crosslinking agent is excluded).
(8) A patch preparation comprising a patch of any one of (1) to (7) wherein the adhesive layer further comprises a drug.

## Effect of the Invention

The patch of the present invention can maintain a large amount of an organic liquid component while simultaneously achieving good adhesiveness, less residue and good cohesion, because an adhesive layer contains a synthetic rubber component other than polyisobutylene and solid at 25˚C, a liquid rubber component having a crosslinkable functional group in the molecule of the liquid rubber component and an organic liquid component, the adhesive layer is crosslinked, and the liquid rubber component is contained in the adhesive layer in a given proportion. This cannot be achieved by merely crosslinking the liquid rubber component, or adjusting the number of functional groups of the liquid rubber component or the degree of crosslinking. However, it can be achieved by adding a heterologous rubber component (synthetic rubber component other than polyisobutylene and solid at 25˚C) to the liquid rubber component, and crosslinking the adhesive layer. The thus-obtained patch provides a soft feeling when applied to the skin and causes a little skin irritation when detached from the skin, due to the organic liquid component contained in the adhesive layer.

Particularly unpredictably, such adhesive layer shows good cohesive strength even though it contains an organic liquid component, and reduces the adhesive remaining after peeling off the patch. In addition, such adhesive layer shows sufficient skin adhesiveness, despite the organic liquid component contained therein. Therefore, the patch of the present invention is highly useful.

Furthermore, since the patch of the present invention does not require use of an acrylic polymer, the adhesive layer can suitably contain a chemical component (e.g., certain kind of drugs) poorly compatible with the acrylic polymer due to its low solubility in acrylic polymers and the like. Accordingly, the patch is suitable for a patch preparation since the degree of freedom in selection of such chemical components is high. In such patch preparation, moreover, release of a drug from the adhesive layer can be controlled by the organic liquid component, and the drug can be stably maintained in the adhesive layer.

## Best Mode for Carrying out the Invention

The patch of the present invention comprises a support and an adhesive layer provided on at least one surface of the support. The adhesive layer contains a synthetic rubber component other than polyisobutylene and solid at 25˚C, a liquid rubber component having a crosslinkable functional group in the molecule of the liquid rubber component and

an organic liquid component, and the adhesive layer is crosslinked. Preferably, the adhesive layer is substantially of a type free of water in view of adhesion to the skin and the like.

In the present invention, a liquid rubber component provides a crosslinking point at which the adhesive layer is crosslinked. Since crosslinking affords a network structure, the component functions to impart the adhesive layer with cohesive strength and cohesive force.

The liquid rubber component is not particularly limited as long as it has a crosslinkable functional group in the molecule of the liquid rubber component. Examples thereof include a liquid isoprene rubber, a liquid butadiene rubber, a liquid isobutylene rubber and the like, which may be used alone or in a combination of two or more kinds thereof. A liquid isoprene rubber is preferable as the liquid component since the liquid rubber component should be liquid even if it has a high molecular weight, the adhesive layer should easily maintain a large amount of the organic liquid component, and a soft feeling should be easily imparted to the adhesive layer. The "liquid" in the present specification means flowability at 25°C.

While the weight average molecular weight of the liquid rubber component is not particularly limited as long as the rubber component is liquid, it is preferably 1,000 - 60,000, more preferably 10,000 - 40,000, most preferably 20,000 - 30,000. When it is less than 1,000, the adhesive layer may fail to achieve sufficient cohesive strength, and a large amount of an organic liquid component may not be maintained easily. Although the mechanism thereof is unknown, it is assumed that a synthetic rubber component other than polyisobutylene and solid at 25°C, cannot be easily held in the network structure of the liquid rubber component, since the molecular weight between crosslinking points of the liquid rubber component becomes small.

On the other hand, when it exceeds 60,000, the liquid rubber component may become non-liquid and, in addition, the content uniformity of the adhesive layer is difficult to maintain. That is, the compatibility of the liquid rubber component with other components of the adhesive layer may be difficult to ensure.

The weight average molecular weight in the present specification means the value measured by gel permeation chromatography under the following conditions:

(analysis conditions)

GPC apparatus: HLC8120 (manufactured by Tosoh Corporation)
column: TSK gel GMH-H(S) (manufactured by Tosoh Corporation)
standard: polystyrene
eluent: tetrahydrofuran
flow rate: 0.5 ml/min.
measurement temperature: 40°C
detection means: differential refractometer

The adhesive layer of the patch of the present invention is crosslinked, which is judged as follows. A sample of the adhesive layer is immersed in toluene at ambient temperature for 6 days, and the presence of insoluble fraction is visually confirmed. When an insoluble fraction is present, the adhesive layer is crosslinked.

While the number of crosslinkable functional groups in a molecule of the liquid rubber component is not particularly limited, it is preferably not less than 3. Since the liquid rubber component contains not less than 3 crosslinkable functional groups in the molecule of the liquid rubber component, the liquid rubber component efficiently forms a network structure and the adhesive layer can maintain a large amount of the organic liquid component. For efficient crosslinking of an adhesive layer, the number of the functional groups in a molecule of the liquid rubber component is more preferably not less than 5, still more preferably not less than 8, and most preferably not less than 10. On the other hand, when the number of the functional groups is high, the amount of the crosslinking agent becomes high. Thus, the number of the functional groups in a molecule is preferably not more than 20.

The functional group of a liquid rubber component is not particularly limited, a carboxyl group, a hydroxyl group, an isocyanate group and a maleic anhydride group (that is, a group represented by the following formula (I))

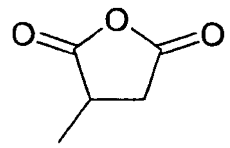

and the like can be mentioned, which may be used alone or in a combination of two or more kinds thereof. Since there are many kinds of reactive crosslinking agents, namely, there are many kinds of reactive functional groups and many kinds of functional groups per molecule, and the reactivity is good, a carboxyl group is preferable.

The number of crosslinkable functional groups in a molecule of a liquid rubber component can be measured

as follows. The total number of crosslinkable functional groups in a liquid rubber component in a sample is determined, which is divided by number average molecular weight of the liquid rubber component to give the number of the crosslinkable functional groups per molecule of the liquid rubber component. When the functional group is a carboxyl group, the number of the carboxyl group in a sample is determined by an acid number measurement method using general potassium hydroxide. The number average molecular weight of the liquid rubber component here means a value determined under the same conditions as for the above-mentioned weight average molecular weight.

The method of crosslinking is not particularly limited, and examples thereof include physical crosslinking by irradiation such as UV or electron beam irradiation and the like, chemical crosslinking treatment using various crosslinking agents and the like.

For convenient crosslinking treatment without an adverse influence on an adhesive layer, a chemical crosslinking treatment wherein the adhesive layer is crosslinked by a crosslinking agent is preferable. Examples of the crosslinking agent include a melamine compound, a compound containing an amino group, a compound containing an epoxy group, a compound containing an isocyanate group, metal oxide, metal halide, metal alkoxide and the like, which may be used alone or in a combination of two or more kinds thereof. In addition, for efficient introduction of crosslinking, the crosslinking agent advantageously contains three or more functional groups in one molecule. In view of the reaction speed, many different kinds and the like, a compound containing an epoxy group, a compound containing an isocyanate group and the like are preferable.

While the amount of the crosslinking agent to be blended is not particularly limited as long as a sufficient crosslinking structure is formed in the adhesive layer, the ratio of the total number (A) of the functional groups of the crosslinking agent in the adhesive layer to the total number (B) of the functional groups of the liquid rubber component in the adhesive layer, $(((A)/(B))\times 100)$[%], is preferably not less than 50%, more preferably not less than 90% and most preferably not less than 300%. Although the upper limit of the value is not particularly limited, it is preferably not more than 400% when many crosslinking agents are added, since the possibility of one molecule of the crosslinking agent blocking the entire functional groups of the liquid rubber to be reacted becomes higher, in which case the crosslinking degree may decrease.

The number of the functional groups of a crosslinking agent can be determined by a general titrimetric method when the functional group is an epoxy group or an isocyanate group, since they easily react with a carboxyl group.

In the present invention, the adhesive layer contains a synthetic rubber component other than polyisobutylene and solid at 25˚C. In the present invention, the synthetic rubber component other than polyisobutylene and solid at 25˚C is added to impart adhesiveness and tackiness to the adhesive layer, or reduce the residue. In the present specification, the "synthetic rubber component other than polyisobutylene" is solid at 25˚C, namely, a synthetic rubber component other than polyisobutylene and free of flowability.

In the present invention, a synthetic rubber component solid at 25˚C, which is other than polyisobutylene free of a functional group, is used. However, the synthetic rubber component other than polyisobutylene and solid at 25˚C may contain a small number of functional groups as long as they do not inhibit the effect of the present invention by, for example, adversely affecting the crosslinking reaction of the adhesive layer and the like.

While the synthetic rubber component other than polyisobutylene and solid at 25˚C is not particularly limited, examples thereof include polyisoprene rubber, polybutadiene rubber, butyl rubber, styrene-diene-styrene block copolymer and the like. These may be used alone or in a combination of two or more kinds thereof.

While the polyisoprene rubber is not particularly limited as long as it is solid at 25˚C, it preferably has a weight average molecular weight of 50,000 - 3,000,000, more preferably 1,000,000 - 2,000,000.

While the polybutadiene rubber is not particularly limited as long as it is solid at 25˚C, it preferably has a weight average molecular weight of 50,000 - 1,000,000, more preferably 300,000 - 600,000.

While the butyl rubber is not particularly limited as long as it is solid at 25˚C, it preferably has a weight average molecular weight of 50,000 - 1,000,000, more preferably 200,000 - 500,000.

While the styrene-diene-styrene block copolymer is not particularly limited as long as it is solid at 25˚C, it is preferably a styrene-isoprene-styrene block copolymer (SIS) in view of its high compatibility with a liquid rubber component, particularly a liquid isoprene rubber, and good adhesiveness. The weight average molecular weight of the styrene-diene-styrene block copolymer is preferably 50,000 - 1,000,000, more preferably 100,000 - 500,000.

When the weight average molecular weight of the above-mentioned synthetic rubber components other than polyisobutylene and solid at 25˚C is smaller than the lower limit, the cohesive strength of the adhesive layer tends to decrease, and the adhesive may remain on the adhesion subject. When it is greater than the upper limit, the compatibility of the adhesive layer with other components may be degraded, or the adhesive layer may be hardened to degrade the adhesiveness.

The viscosity average molecular weight in the present specification is determined by calculating the Staudinger index ($J_0$) by the Schulz-Blaschke equation using the flow time of capillary 1 in a Ubbelohde viscosimeter at 20˚C, and from the following formula using the $J_0$ value:

$$J_0=\eta_{sp}/c(1+0.31\eta_{sp})\ cm^3/g\ \text{(Schulz-Blaschke equation)}$$

$$\eta_{sp}=t/t_0-1$$

t: flow time of solution (by Hagenbach-Couette Correction)
to: flow time of solvent (by Hagenbach-Couette Correction)
c: concentration of solution ($g/cm^3$)

$$J_0=3.06\times10^{-2}\ \overline{Mv}^{0.65}$$

$\overline{Mv}$: viscosity average molecular weight

The proportion of the total weight of the liquid rubber component and the synthetic rubber component other than polyisobutylene and solid at 25˚C is preferably 10 - 60 wt%, more preferably 20 - 50 wt%, most preferably 30 - 45 wt%, relative to the total weight of the adhesive layer (when the adhesive layer contains a crosslinking agent, the weight of the crosslinking agent is excluded). When it is less than 10 wt%, sufficient adhesive property may not be achieved, and when it exceeds 60 wt%, a sufficient soft feeling and a sufficient reduction of skin irritation may not be achieved, since the amount of the organic liquid component that can be contained therein decreases.

In this case, the aforementioned liquid rubber component is preferably contained in a proportion of not more than 40 wt%, more preferably not more than 30 wt%, most preferably not more than 27 wt%, relative to the total weight of the liquid rubber component and the synthetic rubber component other than polyisobutylene and solid at 25˚C. When it exceeds 40 wt%, the compatibility of the liquid rubber component with other components of the adhesive layer is unbalanced, possibly causing phase separation. While the lower limit is not particularly limited, it is preferably not less than 10 wt% since the adhesive layer is sufficiently crosslinked.

In the present invention, the organic liquid component is used for the purpose of plasticizing the adhesive layer to impart a soft feeling or, when a chemical component (e.g., a drug etc.) is contained in the adhesive layer, facilitating transfer of the chemical component in the adhesive layer, and the like. Such organic liquid component is not particularly limited as long as it is compatible with other components of the adhesive layer. Examples thereof include fats and oils such as olive oil, castor oil, lanolin and the like, hydrocarbons such as squalene and liquid paraffin, fatty acid esters such as fatty acid alkylester and the like, higher fatty acids such as oleic acid and caprylic acid, pyrrolidones such as N-methylpyrrolidone and N-dodecylpyrrolidone, sulfoxide such as decylmethylsulfoxide and the like, which may be used alone or in a combination of two or more kinds thereof.

As in the below-mentioned patch preparation, when the adhesive layer contains a drug, a fatty acid ester is preferably used since it promotes transdermal absorption of the drug. Examples thereof include fatty acid alkyl diesters such as dioctyl phthalate, diisopropyl adipate, diethyl sebacate and the like, and fatty acid alkyl monoesters and the like. Of these, a fatty acid alkyl monoester is preferable since it promotes transdermal absorption of the drug.

When the fatty acid alkyl monoester is comprised of a fatty acid having a carbon number which is more or smaller than necessary, the compatibility with the synthetic rubber component other than polyisobutylene and solid at 25˚C, the liquid rubber component and the like may be degraded, or the fatty acid alkyl monoester may be volatilized in the heating step for the production of a preparation. In addition, a fatty acid alkyl monoester comprised of a fatty acid having a double bond in a molecule may be subject to oxidative decomposition and the like to degrade the preservation stability.

Therefore, as such fatty acid alkyl monoester, a fatty acid alkyl ester, which is comprised of a saturated or unsaturated higher fatty acid preferably having a carbon number of 12 - 16, more preferably 12 - 14, and a saturated or unsaturated lower monovalent alcohol preferably having a carbon number of 1 - 4, is preferably employed. Examples of such higher fatty acid preferably include lauric acid (C12), myristic acid (C14), palmitic acid (C16), particularly myristic acid and palmitic acid. Furthermore, examples of such lower monovalent alcohol include methyl alcohol, ethyl alcohol, propyl alcohol and butyl alcohol, which are not limited to straight chain alcohols and may be branched alcohols, with particular preference given to isopropyl alcohol. Accordingly, most preferable fatty acid alkyl esters are isopropyl myristate and isopropyl palmitate.

The organic liquid component is preferably contained in a proportion of not less than 10 wt%, more preferably 10 - 50 wt%, still more preferably 15 - 50 wt%, yet more preferably 20- 40 wt%, most preferably 25 - 35 wt%, relative to the total weight of the adhesive layer (when the adhesive layer contains a crosslinking agent, the weight of the

crosslinking agent is excluded). When it is less than 10 wt%, a soft feeling during application to the skin is impaired, and irritation to the skin may occur on peeling off from the skin. In the case of the below-mentioned patch preparation containing a drug in the adhesive layer, the solubility, releaseability, skin permeability and the like of the drug as preparation characteristics may be unsatisfactory. On the other hand, when it exceeds 50 wt%, the adhesive may remain on the skin as a result of decreased cohesive strength, and also, expression of adhesive property such as tackiness and the like may be difficult.

The adhesive layer preferably contains a tackifier to achieve good tackiness. Examples of such tackifier include polybutene, rosin resin, terpene resin, petroleum resin, cumarone resin, alicyclic saturated hydrocarbon resin and the like, which may be used alone or in a combination of two or more kinds thereof. The tackifier is preferably contained in a proportion of 10 - 55 wt%, more preferably 20 - 50 wt%, most preferably 30 - 45 wt%, relative to the total weight of the adhesive layer (when the adhesive layer contains a crosslinking agent, the weight of the crosslinking agent is excluded). When the proportion of the tackifier is less than 10 wt%, the tackiness may be poor, and when it exceeds 55 wt%, the adhesive layer may unpreferably show a propensity toward destruction. The tackifier to be used in the present invention preferably has a softening point of 70 - 160˚C.

The adhesive layer may contain, as an optional component, other additive (e.g., surfactants such as glycerol fatty acid ester, sorbitan fatty acid ester and the like, high boiling point organic solvents such as dimethyl sulfoxide, N-methylpyrrolidone and the like, absorption promoter such as pyrrolidone carboxylate and the like etc.), acrylic polymer, other rubber components and the like, as long as it does not inhibit the effect of the present invention. The additive is preferably contained in a proportion of 1 - 10 wt% relative to the total weight of the adhesive layer (when the adhesive layer contains a crosslinking agent, the weight of the crosslinking agent is excluded). The thickness of the adhesive layer is generally 20 - 300 μm, preferably 30 - 180 μm.

While the support to be used in the present invention is not particularly limited, a support substantially impermeable to the adhesive layer components, namely, one that does not permit an adhesive layer component to penetrate the support and be lost from the back face to decrease the content is preferable.

As a support, for example, a single film or a laminate film of polyester (e.g., polyethylene terephthalate), nylon, saran (registered trade mark), polyethylene, polypropylene, polyvinyl chloride, ethylene-ethyl acrylate copolymer, polytetrafluoroethylene, surlyn (registered trade mark), metal foil and the like, and the like can be used.

To improve the adhesion force (anchor force) between the support and the adhesive layer, the support is preferably a laminate film of a non-porous plastic film and a porous film, both composed of the above-mentioned materials. In this case, the adhesive layer is preferably formed on the porous film side.

As the porous film, a film capable of improving the anchor force to the adhesive layer is employed. Specifically, paper, woven fabric, non-woven fabric, mechanically perforated sheet and the like are used. Among these, paper, woven fabric and non-woven fabric are particularly preferable from the aspects of handleability and the like.

To improve anchor force, flexibility of the whole patch preparation and operability during adhesion, the porous film has a thickness of 10 - 200 μm. In the case of a thin preparation, a porous film of a plaster type or adhesive tape type preferably having a thickness of 10 - 100 μm is employed. When a woven fabric or non-woven fabric is used as a porous film, the fabric weight is preferably 5 - 30 $g/m^2$, more preferably 6 - 15 $g/m^2$.

Since the patch preparation of the present invention protects the adhesive face of an adhesive layer before use, a release liner is preferably laminated on the adhesive face. The release liner is not particularly limited as long as it ensures sufficient easy-to-release property. Examples thereof include films of polyester, polyvinyl chloride, polyvinylidene chloride, polyethylene terephthalate and the like, laminate films of polyolefin and paper such as high quality paper, glassine paper and the like, high quality paper, glassine paper and the like, and the like, whose face to be in contact with an adhesive layer underwent a peeling treatment by applying a silicone resin, a fluororesin and the like. The thickness of the release liner is generally 10 - 200 μm, preferably 25 - 100 μm.

The form of the patch of the present invention is not particularly limited and may be, for example, a tape, a sheet, a reservoir type and the like.

The present invention also relates to a patch preparation containing a drug in the adhesive layer. The patch preparation of the present invention affords effects similar to those of the above-mentioned patch of the present invention, and moreover, is superior in the solubility, releaseability, skin permeability and the like of the drug.

The drug is not particularly limited, and is preferably one that can be administered to a mammal such as human and the like through the skin, i.e., transdermally absorbable drug. Specific examples of such drug include general anesthetics, hypnotic sedatives, antiepileptic drugs, antipyretic analgesic antiphlogistic drugs, anti-vertigenous drugs, psychoneurotic drugs, topical anesthetics, skeleton muscle relaxants, autonomic drugs, antiepileptic drugs, anti-parkinsonian drugs, anti-histamine drugs, cardiac stimulants, drugs for arrhythmia, diuretic, hypotensive drug, vasoconstrictor, colonary vasodilator, peripheral vasodilators, arteriosclerosis drugs, drugs for circulatory organ, anapnoics, antitussive expectorant, hormone drugs, external drugs for mattery diseases, nalgesic·antipruritic·styptic·antiphogistic drugs, drugs for parasitic dermatic diseases, drugs for arrest of bleeding, gout treatment drugs, drugs for diabetes, drugs for anti-malignant tumor, antibiotic, chemical therapy drugs, narcotic, quit smoking aids and the like.

The proportion of the drug in the adhesive layer is not particularly limited as long as it affords the effect of a transdermally absorbable drug and does not impair the adhesive property of the adhesive. It is preferably 0.1 - 60 wt%, more preferably 0.5 - 40 wt%, relative to the total weight of the adhesive layer (when the adhesive layer contains a crosslinking agent, the weight of the crosslinking agent is excluded). When it is less than 0.1 wt%, the treatment effect may be insufficient, and when it exceeds 60 wt%, stimulation to the skin may occur and the economic aspect may be insufficient.

The patch and patch preparation of the present invention can be produced, for example, by preparing a solution of a composition for forming an adhesive layer comprising a synthetic rubber component other than polyisobutylene and solid at 25°C, a liquid rubber component having a crosslinkable functional group in the molecule of the liquid rubber component and an organic liquid component in a solvent such as toluene and the like, applying the obtained solution of the composition for forming an adhesive layer in a solvent onto a release liner, drying same to form an adhesive layer, and laminating a support on the adhesive layer. Alternatively, for example, a solution of a composition for forming an adhesive layer in a solvent is applied onto a support, and the solvent is evaporated to form an adhesive layer on the support, whereby the patch or patch preparation can be produced.

The patch and the patch preparation of the present invention can be used by detaching a release liner immediately before use and adhering the exposed adhesive face to the skin surface and the like.

**Examples**

The present invention is explained in detail in the following by referring to Production Examples and Experimental Example, which are not to be construed as limitative.

Production Example 1 (patch)

The starting materials were mixed at the mixing ratio shown in Table 1 to give a solution of a composition for forming an adhesive layer in toluene. The solution was applied to a polyethylene terephthalate (PET) film as a release liner such that the film thickness would be 50 μm, and dried to evaporate toluene, whereby an adhesive layer was formed. To the surface of the adhesive layer was adhered a laminate of PET non-woven fabric and PET film as a support, with the PET non-woven fabric surface on the adhesive layer side. This was aged at 60°C for 3 days to give the patches of Examples 1 - 5 and Comparative Examples 1 - 4.
The starting materials and abbreviations thereof used in the Examples and Comparative Examples are as follows:

<Starting materials>

PIB1: polyisobutylene, viscosity average molecular weight 800,000
PIB2: polyisobutylene, viscosity average molecular weight 60,000
IIR: butyl rubber, weight average molecular weight 350,000
IR: polyisoprene rubber, weight average molecular weight 1,490,000
SIS: styrene-isoprene-styrene block copolymer, weight average molecular weight 190,000
BR: polybutadiene rubber, weight average molecular weight 458,000
LIR1: non-functional liquid isoprene rubber, weight average molecular weight 50,000
LIR2: liquid isoprene rubber containing trifunctional maleic anhydride, weight average molecular weight 25,000
LIR2 contains a maleic anhydride group (i.e., the group represented by the aforementioned formula (I)).
LIR3: liquid isoprene rubber containing decafunctional COOH, weight average molecular weight 25,000
crosslinking agent: compound containing tetrafunctional epoxy group, number average molecular weight 366
TF1: tackifier alicyclic saturated hydrocarbon resin, softening point 100°C
IPM: isopropyl myristate

Experimental Example 1

The patches of Examples 1 - 5 and Comparative Examples 1 - 4 were evaluated based on the measurement of the following evaluation items.

(1) General property

(i) Adhesive force: unit: N (width 12 mm)

A cleaned phenol resin board was used as a test board in a room at 23˚C×60%RH. An adhesive face of a 12 mm-wide test piece was lightly adhered to the board, and pressure-bonded by one reciprocation of a 2 kg pressure bonding roller on the test piece. The test piece was stood still in the same environment for 30 min, and peeled off by a tensile tester at an angle of 180˚ and a tensile rate of 300 mm/min. The testing power at that time was determined.

(ii) Cohesive force: unit: min (area 200 $mm^2$)

A cleaned phenol resin board was used as a test board in a room at 23˚C×60%RH. An adhesive face of a 10 mm-wide test piece was lightly adhered to the board, and pressure-bonded by one reciprocation of a 2 kg pressure bonding roller on the test piece. The area of pressure bonding was set to 200 $mm^2$. The test piece was stood still in a cohesive force tester set to 40˚C for 30 min. One end of the test board was stopped such that the test piece would hung perpendicularly, and a load of 300 g was applied to the lower end thereof to determine the time before the board dropped. As for destruction, moreover, it was confirmed that all the test pieces measured at this time were in a cohesive failure mode..

(2) Practical property

(i) Skin irritation:

A panelist touched the surface of the adhesive layer with a finger, and evaluated the layer to be O when physical skin irritation (pain) was weak and × when physical skin irritation was strong.

(ii) Cohesive force:-

When the aforementioned adhesive force was not less than 0.3N, O was marked, and when it was less than 0.3N, × was marked.

(iii) Residue (adhesive residue 1):

Based on the destruction mode of the aforementioned adhesive force, evaluation was made according to the following criteria.

Interface destruction: O
Partial cohesive failure: Δ
cohesive failure: ×
slip stick: ×

(iv) Cohesion (adhesive residue 2):

Based on the aforementioned cohesive force (min), cohesion was evaluated according to the following criteria.

not less than 1.8 min: ○
not less than 0.5 and less than 1.8 min: Δ
not less than 0.2 min and less than 0.5 min: ×

The lowest value is 0.2 min.
The results are shown in Table 1.
【Table 1】

| | | PIB | | synthetic rubber component excluding PIB | | | | liquid rubber component | | | cross-linking agent | tackifier | organic liquid component | adhesive force | | cohesive force | | skin irritation | adhesiveness | residue | cohesion |
|---|---|---|---|---|---|---|---|---|---|---|---|---|---|---|---|---|---|---|---|---|---|
| | | PIB1 | PIB2 | BR | IR | IIR | SIS | LIR1 | LIR2 | LIR3 | functional group proportion | | IPM | N/12 mm | destruction | min | destruction | | | | |
| Example | 1 | | | 25 | | | | | | 10 | 100% | 40 | 25 | 0.81 | K | 7.0 | G | ○ | ○ | ○ | ○ |
| | 2 | | | | 25 | | | | | 10 | 100% | 40 | 25 | 0.37 | K | 4.1 | G | ○ | ○ | ○ | ○ |
| | 3 | | | | | 25 | | | | 10 | 100% | 40 | 25 | 0.52 | K | 0.5 | G | ○ | ○ | ○ | △ |
| | 4 | | | | | | 25 | | | 10 | 100% | 40 | 25 | 1.98 | K | 1.8 | G | ○ | ○ | ○ | ○ |
| | 5 | | | | 25 | | | | 10 | | 100% | 40 | 25 | 0.33 | K | 3.9 | G | ○ | ○ | ○ | ○ |
| Comparative Example | 1 | | | 35 | | | | | | | 0% | 40 | 25 | 0.56 | G | 0.6 | G | ○ | ○ | × | △ |
| | 2 | 34 | | | | | | | | | 0% | 33 | 33 | 0.20 | K | 2.2 | G | ○ | × | ○ | ○ |
| | 3 | | 13 | | 27 | | | | | 7 | 100% | 53 | | 5.00 | Slip | 5.7 | G | × | ○ | × | ○ |
| | 4 | 20 | 10 | | | | | 5 | | | 0% | 40 | 25 | 0.10 | G | 0.2 | G | ○ | × | × | × |

Unit: unless otherwise specified, the ratio relative to the total weight of adhesive layer excluding crosslinking agent (wt%)

proportion of functional group in crosslinking agent (%):ratio of the total number (A) of functional groups of crosslinking agent to the total number (B) of functional groups of liquid rubber component, (((A)/(B))×100)

In the table, K shows interface destruction, G shows cohesive failure, and Slip shows a slip stick.

As is clear from Table 1, in Example 1, the skin irritation, adhesiveness, residue, and cohesion, which show practical property, were all good or within a tolerable range.
In contrast, in Comparative Example 1 wherein LIR3 (functional liquid rubber) was changed to BR (synthetic rubber and solid at 25˚C) and adhesive layer was not crosslinked, unlike Example 1, the residue remained and cohesion also decreased. In Comparative Example 2 wherein BR was changed to PIB1, the organic liquid component was increased and the tackifier was decreased, unlike Comparative Example 1, the residue and cohesion were improved but the adhesiveness was defective. The results show that concurrent use of a synthetic rubber component solid at 25˚C and a functional liquid rubber component in an adhesive layer, and crosslinking of the adhesive layer are effective for the improvement of the residue and cohesion.

Furthermore, in Examples 2, 3 and 4 wherein BR of Example 1 was changed to IR, IIR and SIS, respectively, the skin irritation, adhesiveness, residue, and cohesion, which show practical property, were all good or within a tolerable range. Particularly, Example 4 using SIS was most preferable since the properties were well-balanced.

Moreover, Example 5 wherein LIR3 of Example 2 was changed to LIR2 (trifunctional liquid rubber component) showed good results such as improved cohesion and the like. This shows that the liquid rubber component preferably has at least 3 functional groups in its molecule.
Furthermore, Comparative Example 3 containing almost the same amount of IR as in Example 2 IR and LIR3, and also PIB2, the residue was defective even when the amount of the tackifier was increased and the organic liquid component was omitted. This suggests that IR is more preferable for a patch than PIB. Moreover, since Comparative Example 3 was free of an organic liquid component, skin irritation occurred.

Production Example 2

Example A

In the same manner as in Example 1 except that 25 wt% of IR in Example 2 is changed to 24 wt% of IR and 1 wt% of a drug (indomethacin), a patch preparation of Example A is produced. The patch preparation of Example A shows good practical properties of skin irritation, adhesiveness and adhesive residue, as does the patch of Example 1.

## Claims

1. A patch comprising a support and an adhesive layer provided on at least one surface of the support, wherein the adhesive layer comprises a synthetic rubber component, other than polyisobutylene, which is solid at 25˚C; a liquid rubber component having a crosslinkable functional group in the molecule of the liquid rubber component, and an organic liquid component; wherein the adhesive layer is crosslinked, and the liquid rubber component is contained in a proportion of less than 30 wt% relative to the total weight of the synthetic rubber component solid at 25˚C and the liquid rubber component.

2. A patch according to claim 1, wherein the adhesive layer further comprises a tackifier.

3. A patch according to claim 1 or claim 2, wherein the liquid rubber component is a liquid isoprene rubber.

4. A patch according to any one of claims 1 to 3, wherein the liquid rubber component contains, in the molecule of the liquid rubber component, 3 or more crosslinkable functional groups.

5. A patch according to any one of claims 1 to 4, wherein the synthetic rubber component solid at 25˚C comprises at least one kind selected from a polyisoprene rubber, a polybutadiene rubber and a styrene-diene-styrene block copolymer.

6. A patch according to any one of claims 1 to 5, wherein the adhesive layer is crosslinked with a crosslinking agent, and the ratio of the total number (A) of the functional groups of the crosslinking agent in the adhesive layer to the total number (B) of the functional groups of the liquid rubber component in the adhesive layer, $(((A)/(B))\times100)[\%]$, is not less than 50%.

7. A patch according to any one of claims 1 to 6, wherein the organic liquid component is contained in a proportion of not less than 10 wt% relative to the total weight of the adhesive layer (provided that when the adhesive layer contains a crosslinking agent, the weight of the crosslinking agent is excluded).

8. A patch preparation comprising a patch according to any one of claims 1 to 7, wherein the adhesive layer further comprises a drug.

## Patentansprüche

1. Pflaster, umfassend einen Träger und eine Klebstoffschicht, die sich auf wenigstens einer Fläche des Trägers befindet, wobei die Klebstoffschicht Folgendes umfasst: eine von Polyisobutylen verschiedene Synthesekautschukkomponente, die bei 25 ˚C fest ist; eine flüssige Kautschukkomponente, die eine vernetzungsfähige funktionelle Gruppe im Molekül der flüssigen Kautschukkomponente aufweist, und eine organische Flüssigkeitskomponente; wobei die Klebstoffschicht vernetzt ist und die flüssige Kautschukkomponente in einem Anteil von weniger als 30 Gew.-% enthalten ist, bezogen auf das Gesamtgewicht von bei 25 ˚C fester Synthesekautschukkomponente und flüssiger Kautschukkomponente.

2. Pflaster gemäß Anspruch 1, wobei die Klebstoffschicht weiterhin einen Klebrigmacher umfasst.

3. Pflaster gemäß Anspruch 1 oder 2, wobei die flüssige Kautschukkomponente ein flüssiger Isoprenkautschuk ist.

4. Pflaster gemäß einem der Ansprüche 1 bis 3, wobei die flüssige Kautschukkomponente im Molekül der flüssigen Kautschukkomponente 3 oder mehr vernetzungsfähige funktionelle Gruppen enthält.

5. Pflaster gemäß einem der Ansprüche 1 bis 4, wobei die bei 25 ˚C feste Synthesekautschukkomponente wenigstens eine Art umfasst, die aus einem Polyisoprenkautschuk, einem Polybutadienkautschuk und einem Styrol-Dien-Styrol-Blockcopolymer ausgewählt ist.

6. Pflaster gemäß einem der Ansprüche 1 bis 5, wobei die Klebstoffschicht mit einem Vernetzungsmittel vernetzt ist und das Verhältnis der Gesamtzahl (A) der funktionellen Gruppen des Vernetzungsmittels in der Klebstoffschicht zur Gesamtzahl (B) der funktionellen Gruppen der flüssigen Kautschukkomponente in der Klebstoffschicht, $(((A)/(B))\times 100)$ [%], nicht kleiner als 50% ist.

7. Pflaster gemäß einem der Ansprüche 1 bis 6, wobei die organische Flüssigkeitskomponente in einem Anteil von nicht weniger als 10 Gew.-% enthalten ist, bezogen auf das Gesamtgewicht der Klebstoffschicht (mit der Maßgabe, dass das Gewicht des Vernetzungsmittels ausgeschlossen ist, wenn die Klebstoffschicht ein Vernetzungsmittel enthält).

8. Pflasterpräparat, das ein Pflaster gemäß einem der Ansprüche 1 bis 7 umfasst, wobei die Klebstoffschicht weiterhin einen Wirkstoff umfasst.

## Revendications

1. Patch comprenant un support et une couche adhésive placée sur au moins une surface du support, dans lequel la couche adhésive comprend un composant caoutchouc synthétique, autre que le polyisobutylène, qui est solide à 25˚C, un composant caoutchouc liquide comprenant un groupe fonctionnel réticulable dans la molécule du composant caoutchouc liquide, et un composant liquide organique ; dans lequel ladite couche adhésive est réticulée, et le composant caoutchouc liquide est contenu dans une proportion inférieure à 30 % en poids par rapport au poids total du composant caoutchouc synthétique solide à 25˚C et du composant caoutchouc liquide.

2. Patch selon la revendication 1, dans lequel la couche adhésive comprend en outre un agent poisseux.

3. Patch selon la revendication 1 ou la revendication 2, dans lequel le composant caoutchouc liquide est un caoutchouc d'isoprène liquide.

4. Patch selon l'une quelconque des revendications 1 à 3, dans lequel le composant caoutchouc liquide comprend, dans la molécule du composant caoutchouc liquide, 3 groupes fonctionnels réticulables ou plus.

5. Patch selon l'une quelconque des revendications 1 à 4, dans lequel le composant caoutchouc synthétique solide à 25˚C comprend au moins un type choisi parmi un caoutchouc de polyisoprène, un caoutchouc de polybutadiène

et un copolymère séquencé de styrène-diène-styrène.

6. Patch selon l'une quelconque des revendications 1 à 5, dans lequel la couche adhésive est réticulée avec un agent de réticulation, et le rapport du nombre total (A) de groupes fonctionnels de l'agent de réticulation dans la couche adhésive au nombre total (B) de groupes fonctionnels du composant caoutchouc liquide dans la couche adhésive, ((A)/(B)x100) [%], n'est pas inférieur à 50 %.

7. Patch selon l'une quelconque des revendications 1 à 6, dans lequel le composant liquide organique est compris dans une proportion non inférieure à 10 % en poids par rapport au poids total de la couche adhésive (à condition que lorsque la couche adhésive comprend un agent de réticulation, le poids de l'agent de réticulation soit exclu).

8. Préparation de patch comprenant un patch selon l'une quelconque des revendications 1 à 7, dans laquelle la couche adhésive comprend en outre un médicament.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 10151185 A
- JP 3127727 A
- EP 0781553 A
- EP 1970079 A